# EUROPÄISCHE PATENTSCHRIFT

(11) **EP 0 602 412 B1**
(45) Veröffentlichungstag und Bekanntmachung des Hinweises auf die Patenterteilung: **28.08.1996**
(21) Anmeldenummer: 93118796.7
(22) Anmeldetag: 23.11.1993
(51) Int. Cl.: A61B 17/06

(54) **Nahtmaterialpackung**
Suture package
Emballage de suture

(30) Priorität: 04.12.1992 DE 4240831
(43) Veröffentlichungstag der Anmeldung: 22.06.1994
(73) Patentinhaber: B. BRAUN MELSUNGEN AG, D-34212 Melsungen (DE)
(72) Erfinder: Brandau, Rolf, Dipl.-Ing., D-34576 Homberg (DE)
(74) Vertreter: Selting, Günther, Dipl.-Ing.

(56) Entgegenhaltungen:
- EP-A- 0 458 432
- EP-A- 0 521 170
- DE-A- 2 532 992
- US-A- 4 284 194

## Beschreibung

Die Erfindung betrifft eine Nahtmaterialpackung, beispielsweise für chirurgisches Nahtmaterial, wobei das Nahtmaterial in einer Faltkarte derart enthalten ist, daß es auf einfache Weise kontrolliert herausgezogen werden kann.

Aus DE 25 32 992 C2 ist eine Nahtmaterialpackung bekannt, bei der die Faltkarte aus drei langgestreckten, nebeneinander angeordneten und durch längslaufende Faltlinien voneinander getrennten Faltplatten besteht. Diese Faltplatten werden übereinandergeklappt und in gefaltetem Zustand in eine flache Hülle eingefügt, die an ihren Rändern verschweißt wird. Für die Fadenentnahme wird die Hülle beginnend an einer Längskante aufgerissen, wodurch ein Teil der obersten Platte der Faltkarte ebenfalls abreißt und einen Abschnitt des Fadens freilegt, wobei das Fadenende an einer Lasche einer darunterliegenden Platte festgehalten wird. Zum Entnehmen des Fadens muß das Fadenende oder die daran befestigte Nadel aus der Fixierlasche herausgezogen werden. Dies ist umständlich, insbesondere, wenn die Fadenentnahme mit einer Hand erfolgt, die einen Operationshandschuh trägt.

Der Oberbegriff des Patentanspruchs 1 geht aus von einer Nahtmaterialpackung nach EP 0 458 432 A1. Bei dieser Packung besteht die Faltkarte aus vier in einer Reihe nebeneinander angeordneten Platten, nämlich einer Nadelhalteplatte, einer Deckplatte, einer Wickelplatte und einer über die Wickelplatte faltbaren Überfaltplatte. Das mit einer Nadel versehene Fadenende ist in Fixierlaschen der Nadelhalteplatte eingesteckt und ragt bogenförmig über die Nadelhalteplatte hinaus. Nach dem Öffnen einer die gefaltete Faltkarte umgebenden Hülle muß auch hier das Fadenende bzw. die Nadel mit der Hand ergriffen und von den Fixierlaschen befreit werden, bevor der Faden aus der Faltkarte herausgezogen werden kann.

Der Erfindung liegt die Aufgabe zugrunde, eine Nahtmaterialpackung zu schaffen, die einerseits beim Einlegen des Fadens während des Wickelvorganges das Fadenende sicher festhält und den Fadenwickel nach dem Wickelvorgang fixiert, und andererseits das Entnehmen des Fadens durch Ergreifen des Fadenendes erleichtert.

Die Lösung dieser Aufgabe erfolgt erfindungsgemäß mit den im Patentanspruch 1 angegebenen Merkmalen.

Bei der erfindungsgemäßen Nahtmaterialpackung überragt die Fadenhalteplatte mit ihrer Fixierlasche die Basisplatte, und beim Aufreißen der Hülle wird die Fadenhalteplatte um eine Längskante der Basisplatte herum verschwenkt, wobei das Fadenende des zwischen Wickelplatte und Überfaltplatte gehaltenen Fadens aus der Fixierlasche herausgleitet. Dadurch, daß das Fadenende an der schwenkbaren Fadenhalteplatte herausziehbar eingeklemmt ist, löst es sich beim Verschwenken der Fadenhalteplatte und steht dann frei von dem noch gefalteten Teil der Faltkarte ab. Das Fadenende wird somit dem Benutzer frei vorstehend angeboten, ohne daß es erst aus einer Fixierung herausgezogen werden müßte. Ein wesentlicher Vorteil besteht darin, daß das Fadenende während des Wickelns des Fadens von der Fadenhalteplatte festgehalten wird, wobei der Faden auf der Wickelplatte maschinell verlegt wird. Anschließend wird die Überfaltplatte über die Wickelplatte gefaltet, um den Fadenvorrat festzuhalten. Dann wird die Wickelplatte auf die eine Seite und die Fadenhalteplatte auf die andere Seite der Basisplatte umgefaltet, wobei die Fadenhalteplatte über das Ende der Basisplatte hinausragt. Während des Faltens sind somit der Fadenvorrat und das Fadenende fixiert. Im gefalteten Zustand wird die Faltkarte in die Hülle eingeführt und die Hülle wird zugeschweißt. Beim Aufreißen der Hülle wird die Fadenhalteplatte in bezug auf den Rest der Faltkarte seitlich verschwenkt, wobei das Fadenende sich aus der Fixierlasche löst und zum Ergreifen frei absteht.

Die Fadenhalteplatte ist zweckmäßigerweise unter eine Faltplatte gefaltet, welche an die der Wickelplatte abgewandten Längskante der Basisplatte angrenzt. Diese Faltplatte wird unter die Basisplatte gefaltet, so daß die Fixierlasche sich an der der Basisplatte zugewandten Seite der Fadenhalteplatte befindet, jedoch über die Basisplatte hinausragt. In der Basisplatte kann ein geschlitztes Loch vorgesehen sein, um den Durchgang des Fadens durch die Basisplatte zu ermöglichen. Ein wesentlicher Vorteil besteht darin, daß der Fadenverlauf bei zusammengelegter Faltkarte ungeknickt ist, so daß beim Herausziehen des Fadens auch keine wesentliche Reibung an Kanten auftritt.

In der Faltkarte ist der Fadenwickel fixiert und der Faden gegen mechanische Beschädigung geschützt. Die Faltplatte und die Fadenhalteplatte haben vorzugsweise eine schräg (unter 45°) zur Basisplatte verlaufende Faltlinie, die bei gefalteter Faltkarte eine den Aufreißvorgang der Hülle steuernde Knicklinie bildet. Beim Aufreißen der Hülle wird die Faltkarte überhaupt nicht beschädigt oder gar zerstört.

Im folgenden wird unter Bezugnahme auf die Figuren ein Ausführungsbeispiel der Erfindung näher erläutert.

Es zeigen:
- Fig. 1: die Faltkarte im auseinandergefalteten Zustand,
- Fig. 2: eine Einzelheit aus Fig. 1 mit Angabe der Umfaltrichtungen der Fadenhalteplatte und der Faltplatte,
- Fig. 3: eine Ansicht der Faltplatte nach dem Wickelvorgang,
- Fig. 4: das restliche Umfalten der Faltplatte,
- Fig. 5: die fertig gefaltete Faltplatte,
- Fig. 6: das Einbringen der Faltplatte in die Hülle und
- Fig. 7: das Aufreißen der Hülle zum Entnehmen des Fadens.

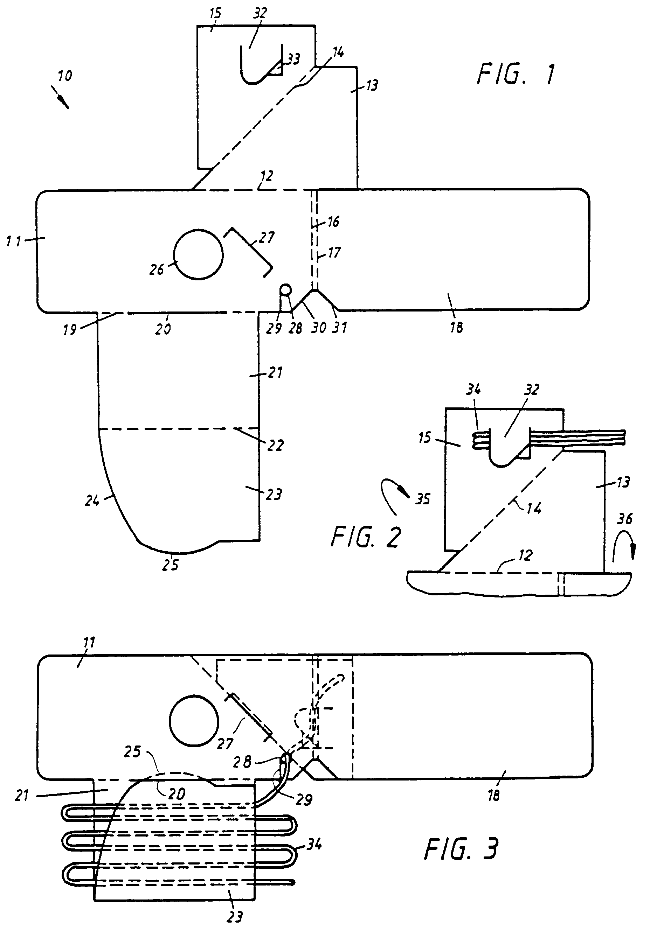

Die in Fig. 1 dargestellte Faltkarte 10 besteht aus einem einstückigen Zuschnitt aus festem Papier oder Karton. Sie weist eine rechteckige, länglich gestaltete Basisplatte 11 auf, deren eine Längskante 12 in Form einer Faltlinie in eine Faltplatte 13 übergeht. Die Faltplatte 13 erstreckt sich über die eine Hälfte der Basisplatte 11 und sie überragt die Basisplatte 11 in Längsrichtung. Die Faltplatte 13 wird durch eine schräg unter 45° zur Längsrichtung der Basisplatte 11 verlaufende Faltlinie 14 begrenzt, deren Verlängerung auf die Längskante 12 der Basisplatte stößt, und an die sich die Fadenhalteplatte 15 anschließt. Die Fadenhalteplatte 15 ist deckungsgleich mit der Faltplatte 13, so daß sie unter die Faltplatte gefaltet werden kann. Beide Platten 13 und 15 sind im wesentlichen dreieckförmig.

An das der Faltplatte 13 benachbarte Ende der Basisplatte 11 schließt sich über zwei parallele Faltlinien 16,17 eine Deckplatte 18 an, die etwa die gleiche Größe hat wie die Basisplatte und über diese gefaltet werden kann.

An der der Längskante 12 abgewandten Längskante der Basisplatten 11 befindet sich eine Faltlinie 19 mit einem Einsteckschlitz 20. An die Faltlinie 19 schließt sich die Wickelplatte 21 an, die an ihrer gegenüberliegenden Kante durch eine Faltlinie 22 begrenzt ist, an welche sich die Überfaltplatte 23 anschließt. Die Wickelplatte 21 und die Überfaltplatte 23 sind kürzer als die Basisplatte 11, die diese beiden Platten 21 und 23 an beiden Enden überragt. Die Überfaltplatte 23 ist im wesentlichen deckungsgleich mit der Wickelplatte 21, jedoch weist sie an einer Kante eine Abrundung 24 und an ihrer äußeren Längskante eine Lasche 25 auf, die in den Einsteckschlitz 20 eingesteckt werden kann.

Die Basisplatte 11 ist mit einem Zentrierloch 26 versehen, in das ein Zentrierdorn einer Wickelmaschine eingesteckt werden kann, um die Faltkarte 10 in der Wickelmaschine zu zentrieren. Ferner ist an der Basisplatte 10 eine U-förmig ausgestanzte Verriegelungslasche 27 vorgesehen, die schräg unter 45° zur Längsrichtung der Basisplatte verläuft und unter die die von der Faltlinie 14 gebildete Faltkante gesteckt werden kann.

In der Nähe der einen Ecke der Basisplatte 11 ist eine Durchführungsöffnung 28 vorgesehen, die über einen Schlitz 29 in die Längskante hinein ausläuft. Die Durchführungsöffnung 28 befindet sich an der der Faltplatte 13 gegenüberliegenden Ecke. In dieser Ecke der Basisplatte 11 ist eine Abschrägung 30 vorgesehen, und an der angrenzenden Ecke der Deckplatte 18 ist eine Abschrägung 31 vorgesehen. Diese Abschrägungen ermöglichen auch bei nicht genauer Plazierung der gefalteten Faltkarte in der Hülle ein sicheres Aufreißen der Hülle.

An der Fadenhalteplatte 15 ist eine Fixierlasche 32 zum Einklemmen des Fadenendes ausgestanzt. Diese Fixierlasche 32 ist der Basisplatte 11 zugewandt. An einer Kante ist sie durch eine Öffnung 33 begrenzt, die während des Aufreißens der Hülle ein leichtes Herausgleiten des Fadenendes aus der Fixierlasche ermöglicht.

In die Faltkarte 10 wird in dem in Fig. 1 dargestellten Zustand das Fadenende eines Fadens 34 gemäß Fig. 2 unter der Fixierlasche 32 fixiert, so daß dieses Fadenende festgeklemmt wird. In Fig. 2 sind mehrere Fadenenden dargestellt, weil dort das Nahtmaterial aus mehreren parallelen Einzelfäden besteht. Die Fadenhaltekarte 15 wird in Richtung des Pfeils 35 um die Faltlinie 14 herum unter die Faltplatte 13 gefaltet, so daß die Fixierlasche 32 sich mit den Fäden 34 auf der Rückseite befindet. Dann wird die Faltplatte 13 um die Längskante 12 herum in Richtung des Pfeiles 36 unter die Basisplatte 11 gefaltet, und die von der Faltlinie 14 gebildete Kante wird unter die Verriegelungslasche 27 der Basisplatte 11 gesteckt. Die Fixierlasche 32 befindet sich nun mit den Fadenenden auf der Rückseite. Der Faden 34 wird durch den Schlitz 29 in die Durchführungsöffnung 28 eingesteckt und auf der Wickelplatte 21 mäanderförmig verlegt. Dieses Verlegen erfolgt in der Wickelmaschine, die (nicht dargestellte) Dorne aufweist, welche zu beiden Enden der Wickelplatte 21 angeordnet sind. Um diese Dorne wird der Faden 34 herumgelegt, so daß er den in Fig. 3 dargestellten Verlauf annimmt. Nach dem Verlegen des Fadens wird die Überfaltplatte 23 über die Wickelplatte 21 gefaltet und die Lasche 25 wird in den Einsteckschlitz 21 eingesteckt, um den Faden 34 zu sichern.

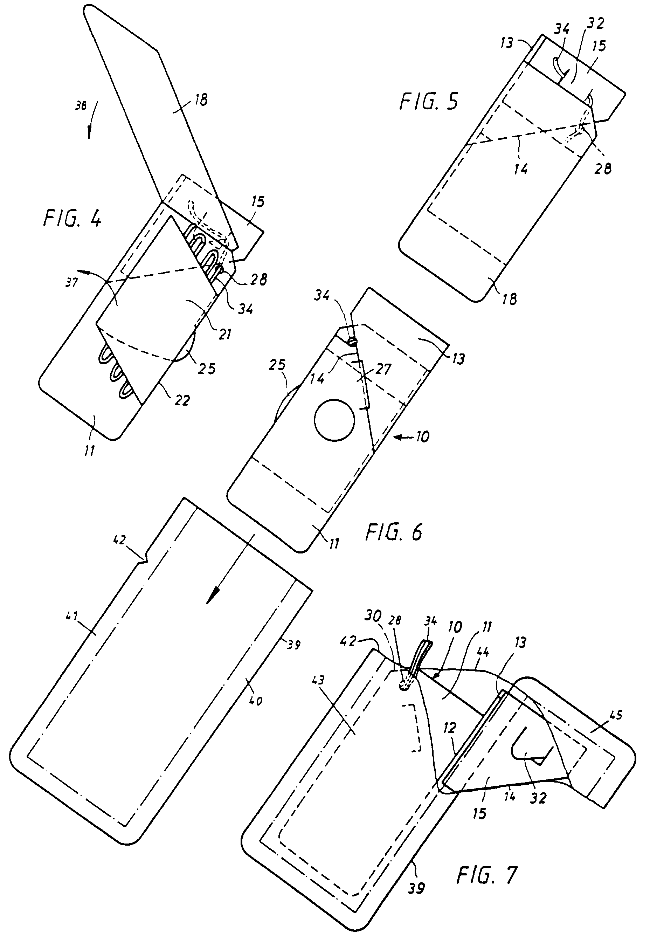

Fig. 4 zeigt das Umklappen der aufeinanderliegenden Wickelplatte 21 und Überfaltplatte 23 um die Faltlinie 22 herum auf die Basisplatte 11, wobei die Fadenhalteplatte 15 (zusammen mit der Faltplatte 13) das eine Ende der Basisplatte 11 überragt. Der Faden läuft ohne Knickung von der nach vorn weisenden Fixierlasche 32 durch die Durchführungsöffnung 28 hindurch in das Innere der von der Wickelplatte 21 und der Überfaltplatte 23 gebildeten Tasche, aus der die Fadenschlingen an beiden Enden herausragen. Das Umklappen der Fadentasche auf die Basisplatte 11 ist mit dem Pfeil 37 bezeichnet. Danach wird die Deckplatte 18 über die von der Wickelplatte und der Überfaltplatte gebildete Tasche gelegt (Pfeil 38).

Fig. 5 zeigt die zusammengefaltete Faltkarte von der Vorderseite, d.h. von derselben Seite, in der sie in den Fign. 1 bis 3 dargestellt ist. Die Faltkarte hat rechteckige, langgestreckte Form und sie ist etwas länger als die Basisplatte 11 bzw. die Deckplatte 18, weil die Fadenhalteplatte 15 mit der Faltplatte 13 die Basisplatte 11 und die Deckplatte 18 an einem Ende überragen. An diesem überstehenden Ende befindet sich die Fixierlasche 32, die das daruntergeklemmte Fadenende festhält. Von dort verläuft der Faden durch die Durchführungsöffnung 28.

In Fig. 6 ist die gefaltete Faltkarte 10 von der Rückseite dargestellt, die von der Basisplatte 11 mit der daruntergefalteten Faltplatte 13 gebildet wird. Die von der schrägen Faltlinie 14 gebildete Faltkante ist unter die Verriegelungslasche 27 der Basisplatte 11 geschoben.

Die Faltkarte 10 wird gemäß Fig. 6 in die Hülle 39 eingeschoben, die aus einem Flachbeutel aus beschichteter Folie besteht, der längs seiner Kanten Siegelnähte 40 und 41 aufweist. Nach dem Einschieben der Faltkarte in die Beutelöffnung wird diese ebenfalls mit einer Siegelnaht verschlossen, so daß die Faltkarte mit dem Nähnahtmaterial vollständig umschlossen ist. Die Hülle 39 weist an ihrer einen Längskante eine Aufreißkerbe 42 auf, an der sie in Höhe der Abschrägungen 30,31 der Faltkarte aufgerissen werden kann. Beim Aufreißen gemäß Fig. 7 reißt die vordere Folie 43 der Hülle 39 längs der von der schrägen Faltlinie 14 gebildeten Faltkante auf, während die hintere Folie 44 unkontrolliert aufreißt. Dabei entsteht eine taschenförmige Aufreißtasche 45 der Folie 39. In dieser Tasche ist der über die Basisplatte 11 und die Deckplatte 18 überstehende Teil der Faltplatte 13 und der Fadenhalteplatte 15 enthalten. Die Faltplatte 13 wird um die Längskante 12 herum aufgeschwenkt. Dabei gleitet das Ende des Fadens 34 aus der Fixierlasche 32 heraus, um sich in der in Fig. 7 gezeigten Stellung anzubieten. Das Ende des Fadens 34 braucht nur noch mit der Hand ergriffen zu werden, um den Faden aus der Faltkarte herauszuziehen. Beim Öffnen der Hülle 39 wird zwar die Hülle zerstört, nicht aber die Faltkarte 10.

## Patentansprüche

1. Nahtmaterialpackung, bestehend aus einer in einer aufreißbaren Hülle (39) enthaltenen Faltkarte (10) mit einer Basisplatte (11), einer daran seitlich angrenzenden Wickelplatte (21) für Nahtmaterial (34), einer über die Wickelplatte (21) faltbaren Überfaltplatte (23) und einer Fadenhalteplatte (15) mit Fixierlasche (32) zum Festhalten eines Fadenendes,
**dadurch gekennzeichnet,**
daß im gefalteten Zustand der Faltkarte (10) die Fadenhalteplatte (15) über die Basisplatte (11) hinaus vorsteht und beim Aufreißen der Hülle (39) um eine Längskante (12) der Basisplatte (11) verschwenkt wird, wobei das Fadenende des zwischen Wickelplatte (21) und Überfaltplatte (23) gehaltenen Fadens aus der Fixierlasche (32) herausgleitet.

2. Nahtmaterialpackung nach Anspruch 1, dadurch gekennzeichnet, daß die Fadenhalteplatte (15) unter eine Faltplatte (13) gefaltet ist, welche an die der Wickelplatte (21) abgewandten Längskante (12) der Basisplatte (11) angrenzt.

3. Nahtmaterialpackung nach Anspruch 2, dadurch gekennzeichnet, daß die Fadenhalteplatte (15) mit der Faltplatte (13) durch eine schräg zu der Längskante (12) der Basisplatte (11) verlaufende Faltlinie (14) verbunden ist.

4. Nahtmaterialpackung nach Anspruch 2 oder 3, dadurch gekennzeichnet, daß die Wickelplatte (21) mit der Überfaltplatte (23) auf die eine Seite der Basisplatte (11) und die Faltplatte (13) mit der umgefalteten Fadenhalteplatte (15) auf die andere Seite der Basisplatte (11) gefaltet ist.

5. Nahtmaterialpackung nach Anspruch 3 oder 4, dadurch gekennzeichnet, daß die schräge Faltlinie (14) zwischen Faltplatte (13) und Fadenhalteplatte (15) an der gefalteten Faltkarte (10) außen liegt und eine Führungsbahn für das Aufreißen der Hülle (39) bildet.

6. Nahtmaterialpackung nach einem der Ansprüche 3 bis 5, dadurch gekennzeichnet, daß die Basisplatte (11) eine schräge Verriegelungslasche (27) zum Einklemmen der Faltlinie (14) von Faltplatte (13) und Fadenhalteplatte (15) aufweist.

7. Nahtmaterialpackung nach einem der Ansprüche 1 bis 6, dadurch gekennzeichnet, daß die Basisplatte (11) nahe der Wickelplatte (21) eine geschlitzte Durchführungsöffnung (28) für den Faden (34) aufweist.

8. Nahtmaterialpackung nach einem der Ansprüche 1 bis 7, dadurch gekennzeichnet, daß an das eine Ende der Basisplatte (11) eine Deckplatte (18) angrenzt, die über die Wickelplatte (21) und die Überfaltklappe (23) geklappt ist, und daß die Faltplatte (13) parallel zu der Deckplatte (18) über die Basisplatte (11) hinausragt.

9. Nahtmaterialpackung nach einem der Ansprüche 2 bis 8, dadurch gekennzeichnet, daß die Fadenhalteplatte (15) die Faltplatte (13), bezogen auf ihre gemeinsame Faltlinie (14), einander im wesentlichen deckungsgleich sind.

## Claims

1. A suture material package, comprising a folding card (10) contained in an envelope (39) adapted to be torn open, the folding card (10) comprising a base panel (11), a winding panel (21) for suture material (34) laterally joining the base panel (11), a fold-over panel (23) adapted to be folded into a position over the winding panel (21), and a thread holding panel (15) with a fixing flap (32) for holding the end of a thread,
**characterized in**
that, in the folded condition of the folding card (10), the thread holding panel (15) extends beyond the base panel (11), and, while the envelope (39) is torn open, is pivoted about a longitudinal edge (12) of the base panel (11) while the end of the thread held between the winding panel (21) and the fold-over panel (23) slides out of the fixing flap (32).

2. The suture material package according to claim 1, characterized in that the thread holding panel (15) is folded under a folding panel (13) joining the longitudinal edge (12) of the base panel (11) that is facing away from the winding panel (21).

3. The suture material package according to claim 2, characterized in that the thread holding panel (15) is connected to the folding panel (13) by a folding line (14) oriented obliquely to the longitudinal edge (12) of the base panel (11).

4. The suture material package according to claim 2 or 3, characterized in that the winding panel (21) with the fold-over panel (23) is arranged in a folded position on one side of the base panel (11), and the folding panel (13) with the folded thread holding panel (15) is arranged in a folded position on the other side of the base panel (11).

5. The suture material package according to claim 3 or 4, characterized in that the oblique folding line (14) between the folding panel (13) and the thread holding panel (15) is located externally on the folded folding card (10) and forms a guide path for tearing open the envelope (39).

6. The suture material package according to any one of claims 3 to 5, characterized in that the base panel (11) is provided with an oblique locking flap (27) for clamping the folding line (14) of the folding panel (13) and the thread holding panel (15).

7. The suture material package according to any one of claims 1 to 6, characterized in that the base panel (11) in the vicinity of the winding panel (21) is formed with a slitted passage opening (28) for the thread (34).

8. The suture material package according to any one of claims 1 to 7, characterized in that one end of the base panel (11) is joined by a cover panel (18) folded over the winding panel (21) and the fold-over panel (23), and that the folding panel (13) extends beyond the base panel (11) in parallel to the cover panel (18).

9. The suture material package according to any one of claims 2 to 8, characterized in that the thread holding panel (15) and the folding panel (13) are substantially congruent relative to their common folding line (14).

## Revendications

1. Emballage de matériau de suture constitué d'un carton de pliage (10) contenu dans une enveloppe (39) pouvant être ouverte par déchirement et ayant une plaque de base (11), une plaque d'enroulement (21) y étant limitrophe latéralement, pour le matériau de suture (34), une plaque de repli (23) pouvant être pliée sur la plaque d'enroulement (21) et une plaque de maintien de fil (15) avec une patte de fixation (32) destinée à fixer une extrémité de fil,
caractérisé en ce que, à l'état plié du carton de pliage (10), la plaque de maintien de fil (15) fait saillie au-dessus de la plaque de base (11) et, lors de l'ouverture par déchirement de l'enveloppe (39) elle est pivotée autour d'une arête longitudinale (12) de la plaque de base (11), l'extrémité du fil maintenu entre la plaque d'enroulement (21) et la plaque de repli (23) ressortant en glissant, hors de la patte de fixation (32).

2. Emballage de matériau de suture selon la revendication 1, caractérisé en ce que la plaque de maintien de fil (15) est pliée sous une plaque de pliage (13) qui est limitrophe à l'arête longitudinale (12), opposée à la plaque d'enroulement (21), de la plaque de base (11).

3. Emballage de matériau de suture selon la revendication 2, caractérisé en ce que la plaque de maintien de fil (15) est reliée a la plaque de pliage (13) au moyen d'une ligne de pliage (14) courant obliquement par rapport à l'arête longitudinale (12) de la plaque de base (11).

4. Emballage de matériau de suture selon la revendication 2 ou 3, caractérisé en ce que la plaque d'enroulement (21) est pliée, avec la plaque de repli (23) sur une face de la plaque de base (11), et la plaque de pliage (13) est pliée avec la plaque de maintien de fil (15) repliée sur l'autre face de la plaque de base (11).

5. Emballage de matériau de suture selon la revendication 3 ou 4, caractérisé en ce que la ligne de pliage (14) oblique entre plaque de pliage (13) et plaque de maintien de fil (15) est placée extérieurement sur le carton de pliage (10) plié et constitue une piste de guidage pour le déchirement de l'enveloppe (39).

6. Emballage de matériau de suture selon l'une des revendications 3 à 5, caractérisé en ce que la plaque de base (11) présente une patte de verrouillage (27) oblique destinée à enserrer la ligne de pliage (14) de la plaque de pliage (13) et de la plaque de maintien de fil (15).

7. Emballage de matériau de suture selon l'une des revendications 1 à 6, caractérisé en ce que la plaque de base (11) présente près de la plaque d'enroulement (21) une ouverture de passage (28) fendue destinée au fil (34).

8. Emballage de matériau de suture selon l'une des revendications 1 à 7, caractérisé en ce qu'à une extrémité de la plaque de base (11) est limitrophe une plaque de couverture (18) qui est rabattue sur la plaque d'enroulement (21) et la plaque de repli (23), et en ce que la plaque de pliage (13) fait saillie au-dessus de la plaque de base (11) parallèlement à la plaque de couverture (18).

9. Emballage de matériau de suture selon l'une des revendications 2 à 8, caractérisé en ce que la plaque de maintien de fil (15) et la plaque de pliage (13) sont sensiblement coïncidentes, par rapport à leur ligne de pliage commune (14).
